# FASCICULE DE BREVET EUROPEEN

(11) **EP 3 237 491 B1**
(45) Date de publication et mention de la délivrance du brevet: **30.08.2023**
(21) Numéro de dépôt: 15828357.2
(22) Date de dépôt: 22.12.2015
(51) Int. Cl.: C08G 69/02, C08G 69/04, C08G 69/26, C08G 69/28

(54) **FABRICATION DE POUDRES DE POLYAMIDE PAR AMINOLYSE D'ESTER**
HERSTELLUNG VON POLYAMIDPULVERN DURCH ESTERAMINOLYSE
PRODUCTION OF POLYAMIDE POWDERS BY ESTER AMINOLYSIS

(30) Priorité: 22.12.2014 FR 1463132
(43) Date de publication de la demande: 01.11.2017
(73) Titulaire: Arkema France, 92700 Colombes (FR)
(72) Inventeur: NOGUES, Pierre, 27300 Bernay (FR); CAMMAGE, Geoffroy, 76000 Rouen (FR)
(74) Mandataire: Arkema Patent
(86) Numéro de dépôt international: PCT/FR2015/053706
(87) Numéro de publication internationale: WO 2016/102879

(56) Documents cités:
- WO-A2-2006/051222
- US-A- 3 329 653
- US-A- 3 376 260
- US-A- 3 379 695
- US-A- 3 459 714

## Description

### DOMAINE DE L'INVENTION

La présente invention se rapporte aux procédés de synthèse de poudres de polyamide (PA) à partir d'au moins un diester et d'au moins une diamine et/ou à partir d'au moins un aminoester. La présente invention concerne plus particulièrement un procédé de fabrication de poudres de polyamide par polycondensation. Elle concerne également le polyamide susceptible d'être obtenu par le procédé de l'invention, soit sous forme de dispersion de poudre soit sous forme de poudre libre, et son utilisation.

### ARRIÈRE-PLAN TECHNIQUE

Industriellement, les polyamides sont préparés par polymérisation, soit d'au moins un couple diamine et diacide, soit d'au moins un aminoacide, soit d'un mélange de ces deux types de monomères. Ces polyamides sont utilisés lorsqu'ils sont sous forme de poudre, pour des applications spécifiques telles que les revêtements de surface, la cosmétique et les technologies d'agglomération de poudre par fusion ou frittage provoqué par un rayonnement électromagnétique pour fabriquer des objets.

Le document de brevet WO2008005415 décrit un procédé de synthèse de polyamide de haut poids moléculaire. Cette synthèse consiste en une polymérisation dans une extrudeuse, à très haute température d'au moins 300°C, entre des monomères de type diamine et diacide, voire diester bien que cette technique ne soit pas vérifiée par des exemples dans le cas du diester.

Le document de brevet EP0515553 décrit une résine composée de polyoxamides et de copoly(amide-oxamide), synthétisée par polycondensation en présence de di-n-butyloxalate. La polymérisation est réalisée en deux étapes successives : dans une première étape une pré-polymérisation est conduite dans le toluène, à température ambiante, pour former un précipité blanc, puis le toluène est évaporé. Dans une seconde étape la polymérisation se poursuit à une température de 270°C. Les propriétés macromoléculaires et de perméabilité à l'oxygène de ces polyoxamides et copoly(amide-oxamide)s sont décrites mais il n'est fait aucune mention des caractéristiques du précipité blanc obtenu.

Un problème constaté dans le contexte de la synthèse de polyamides à partir d'ester est celui des réactions secondaires favorisant notamment l'apparition d'amines N-alkylées. Ces produits indésirables perturbent la polycondensation ainsi que la cristallisation des polyamides.

II faut aussi mentionner différents procédés de fabrication de poudres de polyamide connus de l'homme du métier. La plupart de ces procédés comportent deux étapes en réalisant dans un premier temps la polymérisation et dans un deuxième temps la formation des particules, par exemple par broyage.

Des poudres de polyamide peuvent être obtenues par exemple par broyage ou cryobroyage de granulés de polyamide de diamètre moyen initial de l'ordre de 3 mm. Néanmoins ces transformations mécaniques par réduction de taille aboutissent souvent à des particules de forme irrégulière, présentant une large distribution en taille de particules et dont le diamètre moyen est rarement inférieur à 100 µm.

II est également connu de préparer des poudres de polyamide par dissolution puis reprécipitation dans un « bon solvant » du polyamide. Les solvants des polyamides à température ambiante étant très corrosifs, le choix du « bon solvant » se porte souvent sur un alcool qui solubilise le polyamide à haute température ce qui nécessite de travailler sous pression. Les installations doivent tenir la pression et les conditions de sécurité sont strictes.

Le document EP1797141 décrit une technique d'obtention de poudre de polymère thermoplastique par un procédé d'alliage à matrice perdue. Le mélange incompatible de deux polymères, à l'état fondu, conduit à la formation des nodules d'un polymère dans l'autre. Les poudres de polymères thermoplastiques sont alors récupérées par dissolution dans l'eau de la matrice hydrosoluble. Ce procédé réalisé en plusieurs étapes représente ainsi un intérêt industriel limité.

Le document US 3,459,714 décrit un procédé de préparation de polyamide sous une forme finement granuleuse comprenant une première réaction à une température de 20° à 250°C pour former un condensat préliminaire oligoamide de faible poids moléculaire, puis une condensation additionnelle du condensat préliminaire en le chauffant à une deuxième température entre la première température et juste sous la température de fusion du polyamide résultant.

Enfin, il est connu d'obtenir des poudres de polymère tel que le polyamide par polymérisation anionique précipitante de lactames en solution. La polymérisation est réalisée en présence des monomères, d'un solvant des monomères, d'un amorceur, d'un catalyseur, d'un activateur et sous agitation à une température voisine de 100°C. Ce procédé est spécifique aux polyamides et poly(ester-amide)s obtenus à partir de monomères de type lactames et lactones. Suivant ce procédé, il est possible d'obtenir des poudres de taille micrométrique et de forme sphéroïdale. Cependant, il est peu flexible et ne permet qu'une faible diversification de la nature des poudres en fonction des propriétés finales recherchées de la poudre, en faisant varier la nature des monomères par exemple.

II existe donc un besoin de mettre au point un procédé amélioré de fabrication de poudre de polyamide, notamment pouvant s'adapter à une plus large gamme de monomères, y-compris ceux à base d'ester, tout en conservant le contrôle fin de la forme des particules obtenues.

### RÉSUMÉ DE L'INVENTION

L'invention concerne en premier lieu un procédé de fabrication d'une dispersion de poudre de polycondensat, caractérisé en ce qu'il comprend au moins une étape de polycondensation :
i) d'au moins un diester et au moins une diamine, et/ou
ii) d'au moins un aminoester,
sous agitation, dans un solvant apte à solubiliser à la fois la diamine, le diester et/ou l'aminoester mais pas le polyamide qui se forme pendant la polycondensation, à une température comprise dans la gamme de 50°C à 120°C et en présence de la silice, de sorte que l'on obtient un précipité de poudre en dispersion dans le solvant.

Selon un mode de réalisation avantageux de l'invention, ledit procédé de fabrication de poudre comprend en outre une étape de séparation du solvant et de récupération de la poudre.

Avantageusement, le procédé de l'invention est réalisé en présence optionnelle d'un catalyseur de polycondensation, dont la teneur est comprise dans la gamme de 0 à 50%, de préférence de 0,01 à 50%, de préférence de 0,01 à 30%, de préférence de 0,01 à 20%, en moles par rapport au nombre de moles de l'ensemble des réactifs. Par l'ensemble des réactifs on entend le couple diamine.diester et/ou l'aminoester. L'ajout de catalyseur au-delà de 30%, voire même déjà au-delà de 20%, en moles, n'a généralement plus d'effet notable sur le rendement de la réaction, on observe alors un « plafond » de rendement.

Avantageusement de l'invention, ledit au moins un diester est de formule R₁-(CH₂)ₘ-R₂, où m représente un entier allant de 0 à 36, et R₁, R₂ représentent des fonctions esters identiques ou différentes de formule générale COOR₃, où R₃ représente une chaîne alkyle linéaire ou ramifiée saturée ou insaturée de 1 à 5 atomes de carbone et/ou de formule R₁-(C₆H₄)ₙ-R₂, où n représente un entier allant de 1 à 2, et R₁, R₂ représentent des fonctions esters identiques ou différentes de formule générale COOR₃, où R₃ représente une chaîne alkyle linéaire ou ramifiée saturée ou insaturée de 1 à 5 atomes de carbone.

Selon un mode de réalisation avantageux de l'invention, ledit au moins un diester est choisi parmi l'oxalate de méthyle, l'oxalate d'éthyle, l'oxalate de propyle, l'oxalate de butyle, le dibutyle adipate, le dibutyle azélate, le diméthyle sébacate, le dibutyle subérate, le dibutyle isophtalate, le dibutyle sébacate, le dibutyle laurate, et leurs mélanges.

Avantageusement, ladite au moins une diamine est choisie parmi les diamines aliphatiques ayant de 6 à 12 atomes de carbone, ladite diamine pouvant être arylique et/ou cyclique saturée.

Avantageusement, ladite diamine est choisie parmi l'éthylène diamine, l'hexaméthylènediamine, la pipérazine, la tetraméthylène diamine, l'octaméthylène diamine, 1,9 diaminononane, la décaméthylène diamine, la dodécaméthylène diamine, le 1,5 diaminohexane, le 2,2,4-triméthyl-1,6-diamino-hexane, les polyols diamine, l'isophorone diamine (IPD), le méthyl pentaméthylènediamine (MPDM), la bis(aminocyclohéxyl) méthane (BACM), la bis(3-méthyl-4 aminocyclohéxyl) méthane (BMACM), la méthaxylyènediamine, le bis-p aminocyclohexylméthane et la triméthylhexaméthylène diamine, et leurs mélanges.

Avantageusement, ledit au moins un aminoester est de formule R₅-(CH₂)ₚ-R₆, avec p représente un entier allant de 0 à 36, R₅ étant une fonction amine primaire ou secondaire et R₆ étant une fonction ester de formule générale COOR₇, R₇ étant une chaîne linéaire ou ramifiée d'alkyles saturés ou insaturés de 1 à 5 atomes de carbone.

Avantageusement, ledit catalyseur de polycondensation est choisi parmi l'hydrure de sodium, l'hydrure de potassium, le sodium, le stéarate de sodium, l'acide ortho-phosphorique, l'acide stéarique, l'éthanol, le phénol, le méthylate de sodium, l'éthylate de sodium, et leurs mélanges.

Avantageusement, ledit solvant est choisi parmi les alcanes linéaires, les alcanes cycloaliphatiques, les solvants halogénés et leurs mélanges, le solvant présentant de préférence une température d'ébullition supérieure à la température de polymérisation comprise dans la gamme de 30°C à 180°C, de préférence dans la gamme allant de 30°C à 150°C, de préférence de 30 à 100°C, et de préférence au voisinage de 100°C. D'autre part, on préfère utiliser un (ou des) solvant(s) liquide(s) à température ambiante (température comprise dans la gamme de 15 à 25°C).

Avantageusement, ladite polycondensation est caractérisée par une précipitation simultanée sous forme de poudres du polycondensat.

Avantageusement, ladite agitation est de vitesse comprise dans la gamme de 1 à 2000 tr/min, de préférence réalisée au moyen d'un système d'agitation à pale, encore plus préférentiellement au moyen d'un système d'agitation à pale et contre-pale.

L'invention concerne également un polyamide obtenu selon le procédé décrit ci-dessus.

Ladite poudre de polycondensat comprend des particules de poudre libre qui présentent une forme sphéroïdale, un D50 mesuré selon la norme ISO 13320-1:1999 compris dans la gamme de 1 à 200 µm, et comprennent des traces de fins de chaînes esters, de préférence 10 à 4000 meq/kg de fins de chaînes esters, sur le poids de polycondensat.

Avantageusement, le polycondensat comprend au moins un monomère choisi parmi les motifs : 2.9, 2.10, 4.6, 4.T, 6, 6.6, 6.9, 6.10, 6.12, 6.T, 9.2, 10.2, 10.10, 10.12, 10.T, 11, 12, 12.9, 12.10, 12.12, 12.T, et leurs mélanges ayant des motifs alternés ou séquences. Dans ce mode de réalisation T étant l'acide téréphtalique.

Avantageusement, la poudre présente des traces du solvant utilisé pour la dispersion, de préférence de 10 à 10000 ppm de solvant.

Avantageusement, on trouve des traces d'un catalyseur de polycondensation, de préférence de 0 à 50%, de préférence de 0,01 à 30%, de préférence de 0,01 à 20%, en moles par rapport au nombre de moles de polycondensat.

Avantageusement, on trouve des traces de monoalcool, de préférence de 0,01 à 5% en poids par rapport au poids total de la poudre, d'alcool de formule R₃OH et/ou R₇OH, où R₃, R₇ représentent chacun une chaîne alkyle linéaire ou ramifiée saturée ou insaturée de 1 à 5 atomes de carbone.

Avantageusement, ledit polycondensat est un oligomère de masse molaire moyenne en nombre comprise dans la gamme de 300 g/mol à 5000 g/mol.

Avantageusement, ledit polycondensat est un polyamide de masse molaire moyenne en nombre comprise entre 5000 à 30000 g/mol, de préférence comprise dans la gamme de 8000 à 15000 g/mol.

Avantageusement, la poudre de polyamide est utilisée dans les revêtements, les peintures, les compositions anticorrosion, les additifs pour papier, les technologies d'agglomération de poudre par fusion ou frittage provoqué par un rayonnement électromagnétique pour fabriquer des objets, les gels d'électrophorèse, les matériaux composites multicouches, l'industrie de l'emballage, les jouets, le textile, l'automobile et/ou l'électronique, dans les produits cosmétiques, pharmaceutiques ou de parfumerie.

Avantageusement, ladite poudre d'oligomère est utilisée comme synthon réactif ester et/ou amine dans les réactions d'allongement de chaînes de polymère, soit seul soit comme additif dans les technologies d'agglomération de poudre par fusion ou frittage provoqué par un rayonnement électromagnétique pour fabriquer des objets, comme renfort de polyamide ou comme charge organique pour les matériaux composites.

L'invention concerne également une dispersion de poudre comprenant :
- de 0,1 % à 99,9% en poids, de préférence de 0,1 à 30% en poids, de particules de poudre conforme à l'invention,
- de 0,1% à 99,9% en poids, de préférence de 70 à 99,9% en poids, de solvant conforme à l'invention, sur le poids total de la dispersion.

L'invention peut aussi être incluse dans une composition cosmétique et/ou de parfumerie.
Avantageusement, ladite composition cosmétique et/ou de parfumerie comprend :
- de 0,1 à 99,9%, de préférence de 0,1 à 30% en poids, de particules de poudre selon l'invention,
- de 0,1 à 99,9%, de préférence de 70 à 99,9% en poids, d'un milieu acceptable en cosmétique et/ou en parfumerie, sur le poids total de la composition.
Avantageusement, ladite composition est un produit coloré, non coloré ou transparent choisi parmi les produits suivants :
- produits de maquillage pour le visage et le corps humain, tels que fond de teint, crème teintée, poudre libre ou compacte, fard à paupières, mascara, eye liner, rouge à lèvre,
- produits de soins pour le visage et le corps humain, tels que crème, lait, lotion, masque, produit de gommage, produits nettoyants et/ou démaquillants, déodorants, antitranspirants, antiperspirants, produits de rasage, produits d'épilation,
- produits capillaires, tels que shampooings, produits pour la mise en forme des cheveux, produits de maintien de la coiffure, produits antipelliculaires, produits antichute, produits contre la sécheresse des cheveux, teintures capillaires, produits de décoloration,
- produits de parfumerie, tels parfum, lait, crème, poudre libre ou compacte parfumée.

La chimie des esters étant en développement croissant, de plus en plus de produits commerciaux sont proposés, augmentant l'intérêt de la présente invention. Un autre avantage de la présente invention est de réaliser la synthèse de poudre de polyamide à des températures inférieures à celle de l'état de la technique. Ceci à pour conséquence des économies d'énergie et un impact positif sur l'environnement.

La présente invention permet de surmonter plusieurs inconvénients de l'état de la technique. Elle fournit plus particulièrement un procédé de fabrication de polyamide à partir d'au moins un diester et au moins une diamine et/ou d'au moins un aminoester dans lequel la poudre de polyamide obtenue présente des caractéristiques avantageuses et une facilité de synthèse remarquable. Le procédé permet de réaliser à basse température (température inférieure à 200°C) et en milieu dispersé la production de poudre de polyamide présentant une granulométrie contrôlée, une morphologie sphéroïdale et une porosité de surface.

Ce type de réaction pour un couple diamine/diacide n'est pas réalisable par l'homme du métier dans ces conditions à cause de la non-réactivité du couple diamine/diacide dans les conditions propres au procédé de polymérisation précipitante. Le demandeur a découvert que, de façon surprenante, le procédé de polymérisation précipitante est utilisable selon l'invention, grâce à la réactivité des diesters avec les diamines. En effet, la réaction d'aminolyse d'ester permet d'utiliser un procédé de polymérisation précipitante normalement réservé à la polymérisation anionique des lactames en solution. La polycondensation d'au moins un diester et d'au moins une diamine et/ou d'au moins un aminoester donne d'excellents résultats dans une réaction opérée selon l'invention. Il est intéressant de noter que la présence d'un amorceur et d'un activateur habituellement requise dans de telles réactions, n'est pas requise dans la présente invention. Avantageusement, la réaction selon l'invention est donc réalisée de façon beaucoup plus simple qu'avec des lactames, dans des plages de températures aux alentours de 100°C et sous agitation.

### DESCRIPTION DE MODES DE REALISATION DE L'INVENTION

L'invention est maintenant décrite plus en détail et de façon non limitative dans la description qui suit.

En l'absence d'indications contraires, les proportions ou pourcentages indiqués sont en poids.

Par polycondensat au sens de l'invention on entend à la fois un oligomère et/ou un polyamide obtenu par polycondensation.

Par polyamide au sens de l'invention on entend les produits de condensation des aminoesters et/ou des diesters avec les diamines et, en règle générale, tout polymère formé par des motifs reliés entre eux par des groupes amides.

Par oligomère au sens de l'invention, on entend une molécule chimique composée de un à quatre monomères.

Par monomère au sens de l'invention on entend une unité répétitive de l'oligomère ou du polyamide. Le cas où une unité répétitive est constituée de l'association d'un diester avec une diamine est particulier. On considère que c'est l'association d'une diamine et d'un diester, c'est-à-dire le couple diamine.diester (en quantité équimolaire), qui correspond au monomère. Ceci s'explique par le fait qu'individuellement, le diester ou la diamine n'est qu'une unité structurale, qui ne suffit pas à elle seule à former un polymère.

L'invention prévoit de produire une poudre de polycondensat à partir d'au moins un diester et au moins une diamine, et/ou d'au moins un aminoester, tel que définis précédemment.

Selon un mode de réalisation particulier de l'invention, un seul couple diamine diester est utilisé, de façon à former un homopolyamide.

Selon un mode de réalisation préféré de l'invention, deux ou plusieurs couples diamines diesters sont utilisés, de façon à former un copolyamide (CoPA).

Selon un autre mode de réalisation particulier, un seul aminoester présentant un seul indice n est utilisé de façon à former un homopolyamide.

Selon un mode de réalisation encore plus préféré, deux ou plusieurs aminoesters présentant des indices n différents sont utilisés, de façon à former un CoPA.

Selon le procédé de l'invention, on introduit par exemple dans un réacteur le ou les monomère(s) compris dans la gamme de 20 à 75%, de préférence 30%, en poids d'au moins un diester et au moins une diamine et/ou d'au moins un aminoester, dans un solvant de dispersion, sur le poids total de dispersion. Le solvant peut comprendre notamment un ou plusieurs des composés suivants : alcanes linéaires, alcanes cycloaliphatiques, solvants halogénés et leurs mélange. Le solvant comprend de préférence un solvant de type Shellsol. Par solvant de type Shellsol on entend un solvant constitué d'une coupe d'hydrocarbure.

On peut également introduire dans le milieu un catalyseur de polycondensation dont la teneur est comprise dans la gamme de 0 à 50%, de préférence de 0,01 à 50%, de préférence de 0,01 à 30%, de préférence de 0,01 à 20%, en moles par rapport au nombre de moles de l'ensemble des réactifs. L'utilisation d'un catalyseur a un effet avantageux sur le contrôle de la polymérisation et sur la précipitation des poudres. Selon un mode de réalisation avantageux du procédé de l'invention, on peut employer alternativement une catalyse acide et une catalyse basique. De préférence, on utilise une catalyse basique, comprenant de préférence le méthylate de sodium.

La température de réaction est comprise dans la gamme de 50°C à 120°C, de préférence de 80 à 110°C, de préférence égale à 100°C.

Une agitation est appliquée au milieu réactionne, comprise de préférence dans la gamme de 1 à 2000 tours/min, de préférence 150 tours/min, de préférence 310 tours/min. Cette agitation peut être faite par tous systèmes suffisants pour engendrer la dispersion des poudres de PA dans le solvant, y compris par cisaillement.

Selon un mode de réalisation avantageux de l'invention, l'agitation est réalisée au moyen d'un système d'agitation à pale, de préférence au moyen d'un système d'agitation à pale et contre pale.

Cette étape dure par exemple au moins 1 heure, ou au moins 2 heures, ou au moins 3 heures, ou au moins 4 heures, ou au moins 5 heures.

La température est de préférence constante. Alternativement, cette température peut varier par exemple de manière monotone ou cyclique ou par paliers. Une phase de montée en température peut être prévue au début du procédé ou avant. De préférence, celle-ci présente une durée inférieure à 30 minutes, ou inférieure à 20 minutes, ou inférieure à 15 minutes, ou inférieure à 10 minutes.

L'aminolyse d'ester conduit selon le procédé de l'invention à la formation d'un polycondensat. Selon l'invention, l'aminolyse d'ester, par réaction d'au moins une diamine et au moins un diester et/ou d'au moins un aminoester entraîne directement la synthèse de polycondensat.

Des réactions parasitaires peuvent théoriquement être observées, comme une cyclisation du diester, une cyclisation intramoléculaire, une cyclisation intermoléculaire ou une N,N' diméthylation. L'alkylation des amines par les esters, provenant de la compétition des groupes acyl-alkyl (N-alkylation), entraine l'arrêt de la formation du polyamide, car il y a formation permanente d'extrémités de chaînes non réactives. Sans être liés par une quelconque théorie, les inventeurs pensent que, grâce au procédé selon l'invention, seule la dernière réaction est possible à basse température (température inférieure à 200°C), toutes les autres réactions étant liées à une haute température (supérieure ou égale à 200°C). Plus la température de réaction est basse et moins la N-alkylation se produit. Avantageusement, le procédé selon l'invention permet de réduire voire d'éviter les réactions parasitaires.

Simultanément à la polycondensation, se forme une dispersion de poudre dans le solvant. Le milieu réactionnel s'opacifie, marquant le début de la formation d'une poudre, de préférence au bout d'une heure.

La dispersion de poudre peut être conservée dans cet état ou bien la poudre peut être séparée du solvant de dispersion et récupérée. De préférence on récupère ainsi le polycondensat sous forme de poudre.

Le cas échéant, l'étape de séparation peut être réalisée selon toutes techniques de séparation liquide-solide, de préférence par évaporation du solvant.

Divers additifs peuvent être également introduits dans le milieu, notamment des charges organiques, telles que du PA, ou des charges minérales, telles que de la silice ; et/ou des tensio-actifs.

De la silice est ajoutée au milieu réactionnel pour aider à la précipitation en permettant de contrôler la distribution en taille des particules. On peut utiliser une silice hydrophile de 5 µm (Sipernat 320DS), mais on utilise de préférence une silice hydrophobe composée d'agglomérats de taille inférieure au micromètre (Aerosil R972). L'ajout de silice a un effet avantageux sur la taille de la poudre de polycondensat. En l'absence d'une telle charge, la poudre tend à former des agglomérats qui coalescent, et il s'avère dans ce cas difficile, voire impossible, de contrôler la distribution en taille des particules, et donc d'obtenir une poudre de D50 inférieur à 100 µm, ou même de D50 inférieur à 200 µm.

Avantageusement, différents types de tensio-actifs peuvent être ajoutés au milieu réactionnel. Ces tensio-actifs permettent de stabiliser les particules en croissance dans le milieu réactionnel. Ils peuvent être par exemple du stéarate de sodium ou du butanol, de préférence du butanol.

La masse molaire en nombre du polyamide obtenu peut être déterminée par Chromatographie d'Exclusion Stérique, en utilisant comme solvant et éluant l'hexafluoroisopropanol (HFIP), et une détection réfractométrique. La masse molaire en nombre du polyamide obtenu peut également être déterminée par RMN (Résonance Magnétique Nucléaire), ou encore par dosage des extrémités de chaîne. On utilise de préférence la RMN pour déterminer la masse molaire en nombre.

Les caractéristiques des poudres de polyamide, objet de l'invention sont en particulier :
- le diamètre moyen des particules de 1 à 200 µm, de préférence de 1 à 100 µm, encore plus avantageusement de 5 à 60 µm ;
- la répartition granulométrique en volume étroite. La distribution granulométrique en volume des poudres est déterminée selon les techniques habituelles, par exemple à l'aide d'un granulomètre Coulter LS 230, selon la norme ISO 13320-1 :1999. A partir de la distribution granulométrique en volume, il est possible de déterminer le diamètre moyen en volume (« D50 ») ainsi que la dispersion granulométrique (écart-type) qui mesure la largeur de la distribution. C'est l'un des avantages du procédé décrit que de permettre d'obtenir une distribution granulométrique (de dispersion) resserrée ou étroite en volume ;
- la forme sphéroïdale des particules, c'est à dire en forme de sphéroïde, qui a une forme proche de la sphère ;
- la porosité de surface des particules, mesurée par la surface spécifique apparente (appelée aussi SSA). Les particules de l'invention présentent une SSA mesurée selon la méthode BET allant de 1 à 20 m²/g, de préférence de 2 à 10 m²/g, de préférence de 3 à 6 m²/g. La méthode BET (BRUNAUER-EMMET-TELLER) est une méthode connue de l'homme du métier. Elle est notamment décrite dans The journal of the American Chemical Society, vol.60, page 309, février 1938, et correspond à la norme internationale ISO 5794/1. La surface spécifique mesurée selon la méthode BET correspond à la surface spécifique totale, c'est-à-dire qu'elle inclut la surface formée par les pores.

### EXEMPLES

Les exemples suivants ne sont pas selon l'invention.

### Exemple 1- Polymérisation précipitante d'un PA 6.10

On prépare un mélange réactionnel dibutyle sebaçate/hexaméthylène diamine dans un rapport massique 1 :1. Le mélange réactionnel est introduit dans un réacteur en verre à hauteur de 30% massique dans du Shellsol. Du méthanolate de sodium est ajouté au milieu réactionnel à hauteur de 17% molaire par rapport à l'un des monomères. La réaction est effectuée sous agitation à 150 tr/min et chauffée jusqu'à une température de 100°C. La durée de la réaction est de 7 heures.

Le milieu est ensuite filtré pour récupérer la poudre de PA 6.10, puis la poudre est lavée à l'éthanol, puis à l'eau et enfin séchée en étuve à 75°C. La poudre est analysée par RMN ¹H et DSC.

Le rendement massique total est de 72%. Ce rendement massique est calculé en faisant le rapport de la masse de poudre obtenue après lavage et séchage sur la masse de poudre théoriquement attendue en fin de réaction.

La poudre de PA 6.10 obtenue a une masse molaire d'environ 1200 g/mol.

La poudre de PA 6.10 obtenue a une température de fusion (T_{f}) égal à 201 °C et une viscosité de 0,23 (selon méthode ARKEMA : 0,5 g/dl dans métacrésol à 25°C).

Le D50 de la poudre est ensuite mesuré selon la norme ISO 13320-1 :1999. Le diamètre moyen des particules de poudre obtenues est de 80 µm.

**Tableau 1 : Analyse RMN ¹H de l'exemple 1**

| Ratio molaire | Ex.1 |
|---|---|
| amide secondaire | 76,12% |
| Ester | 3,79% |
| Acide | 5,76% |
| amine primaire | 11,60% |
| Alcool | 2,72% |

Les analyses RMN montrent bien la production d'un polyamide à partir d'un diester et d'une diamine.

### Tests : Influence la concentration en catalyseur et du profil de température sur le rendement massique de la synthèse

Des essais ont été réalisés en faisant varier la concentration en catalyseur. Les conditions utilisées sont identiques à celles de l'exemple 1. Les résultats sont présentés dans le tableau suivant (plus le rendement massique est important plus la réaction est efficace).

**Tableau 2 : Influence la concentration en catalyseur sur le rendement**

| **Essai** | **Catalyseur [%mol]** | **Rendement massique** |
|---|---|---|
| 1 | 17 | 72 % |
| 2 | 9 | 20 % |
| 3 | 40 | 72 % |

D'après les rendements massiques obtenus dans les conditions de l'exemple 1, il est possible de conclure qu'il existe un chargement optimal en méthanolate de sodium (catalyseur de la réaction) : environ 17 % mol. En effet, avec moins de catalyseur (9 % mol), la réaction donne un rendement massique inférieur ; et avec plus de catalyseur (40 % mol), il n'y a pas d'amélioration observée par rapport à 17% mol.

Des essais ont été réalisés en faisant varier la température de réaction. Les résultats son présentés dans le tableau suivant (plus le rendement massique est important plus la réaction est efficace).

**Tableau 3 : Influence du profil thermique sur l'action du méthanolate de sodium**

| **Essai** | **Catalyseur [%]** | **Profil thermique** | **Rendement massique de précipitation** |
|---|---|---|---|
| 1 | 17 | 100°C sur 7h | 72% |
| 4 | 17 | 100°C sur 15h | 72% |
| 5 | 17 | 80°C sur 15h | 52% |
| 6 | 17 | 120°C sur 7h | 13% |

D'après les rendements massiques obtenus, il est possible de conclure qu'il existe une température de synthèse optimale : 100°C. En effet, il apparait qu'avec une température plus basse (80°C) ou plus haute (120°C), la réaction est moins efficace.

Des essais ont été réalisés en faisant varier la vitesse d'agitation du milieu réactionnel. Les résultats son présentés dans le tableau suivant (plus le rendement massique est important plus la réaction est efficace).

**Tableau 4 : Influence de la vitesse d'agitation sur la taille des particules**

| **Essai** | **Vitesse d'agitation [tr/min]** | **Diamètre moyen [µm]** |
|---|---|---|
| 1 | 150 | 100 |
| 7 | 310 | 80 |

La vitesse d'agitation a un effet direct sur la taille des particules de poudre de polycondensat, plus l'agitation est rapide plus les particules sont petites.

### Exemple 2- Emulsion huile/eau de la poudre de polycondensat selon l'invention

On prépare une formulation A pour une crème cosmétique, du type huile dans eau, ayant la composition massique suivante :
- Eau : 83,44 %.
- Chlorophénésine : 0,28 %.
- Gomme xanthane : 0,2 %.
- Acrylate d'hydroxyéthyle et copolymère de taurate d'acryloyldiméthyle de sodium : 0,5 %.
- Alcool arachidyle et alcool béhényle et glucoside d'arachidyle : 3,0 %.
- Triglycérides caprylique / caprique : 5,0 %.
- Cyclohexasiloxane : 1,0 %.
- Phénoxyéthanol et éthylhexylglycérine : 0,5 %.
- Antioxydant : 0,08 %.
- Glycérine : 3,0 %.
- Composition selon l'invention : 3,0 %.

On prépare une formulation B pour une poudre cosmétique pressée pour le lissage des imperfections, ayant la composition massique suivante :
- Xyloside octyldodécyle : 5,0 %.
- Isostéarate d'isostéaryle : 3,0 %.
- Talc : 44,7 %.
- Mica : 30,0 %.
- Talc et glutamate de stéaroyle disodium / hydroxyde d'aluminium :5,0 %.
- Cellulose : 5,0 %.
- Acide salicylique : 0,2 %.
- Pigments : 2,1 %.
- Composition selon l'invention : 5,0 %.

Dans les deux formulations précédentes, la composition selon l'invention est fabriquée à partir de particules de copolyamide 6/12.

Les particules présentent un diamètre moyen en volume D50 de 10 µm.

La composition présente un pH compris dans la gamme de 5 à 9, par exemple de 7,5.

## Revendications

1. Procédé de fabrication d'une dispersion de poudre de polycondensat, **caractérisé en ce qu'**il comprend au moins une étape de polycondensation:
i) d'au moins un diester et au moins une diamine,
et/ou
ii) d'au moins un aminoester,
sous agitation, dans un solvant apte à solubiliser à la fois la diamine, le diester et/ou l'aminoester mais pas le polyamide qui se forme pendant la polycondensation, à une température comprise dans la gamme de 50°C à 120°C et en présence de la silice, de sorte que l'on obtient un précipité de poudre en dispersion dans le solvant, on récupère le polycondensat sous forme de poudre.

2. Procédé de fabrication d'une dispersion de poudre selon la revendication 1, **caractérisé en ce qu'**il comprend en outre une étape de séparation du solvant et de récupération de la poudre.

3. Procédé selon l'une quelconque des revendications 1 ou 2, dans lequel ladite polycondensation est réalisée en présence d'un catalyseur de polycondensation dont la teneur est comprise dans la gamme de 0,01 à 50%, de préférence de 0,01 à 30%, en moles par rapport au nombre de moles de l'ensemble des réactifs.

4. Procédé selon l'une quelconque des revendications 1 à 3, dans lequel ledit au moins un diester est de formule :
R₁-(CH₂)ₘ-R₂, où m représente un entier allant de 0 à 36, et R₁, R₂ représentent des fonctions esters identiques ou différentes de formule générale COOR₃, où R₃ représente une chaîne alkyle linéaire ou ramifiée, saturée ou insaturée de 1 à 5 atomes de carbone,
et/ou
R₁-(C₆H₄)ₙ-R₂, où n représente un entier allant de 1 à 2, et R₁, R₂ représentent des fonctions esters identiques ou différentes de formule générale COOR₃, où R₃ représente une chaîne alkyle linéaire ou ramifiée, saturée ou insaturée de 1 à 5 atomes de carbone.

5. Procédé selon l'une quelconque des revendications 1 à 4, dans lequel ladite au moins une diamine primaire ou secondaire est choisie parmi les diamines aliphatiques ayant de 6 à 12 atomes de carbone, la dite diamine pouvant être arylique ou cyclique saturée.

6. Procédé selon l'une quelconque des revendications 1 à 5, dans lequel ledit au moins un aminoester répond à la formule générale R₅-(CH₂)ₚ-R₆, où p représente un entier allant de 0 à 36, R₅ représente une fonction amine primaire ou secondaire, R₆ représente une fonction ester de formule générale COOR₇, où R₇ représente une chaîne alkyle de 1 à 5 atomes de carbone.

7. Procédé selon l'une quelconque des revendications 3 à 6, dans lequel le catalyseur de polycondensation est choisi parmi l'hydrure de sodium, l'hydrure de potassium, le sodium, le stéarate de sodium, l'acide ortho-phosphorique, l'acide stéarique, l'éthanol, le phénol, le méthylate de sodium, l'éthylate de sodium, et leurs mélanges.

8. Procédé selon l'une quelconque des revendications précédentes dans lequel le solvant est choisi parmi les alcanes linéaires, les alcanes cycloaliphatiques, les solvants halogénés et leurs mélanges, le solvant présentant de préférence une température d'ébullition comprise dans la gamme de 30 à 180°C.

9. Procédé selon l'une quelconque des revendications précédentes dans lequel la polycondensation est **caractérisée par** une précipitation simultanée sous forme de poudres de polycondensat.

10. Procédé selon l'une des revendications précédentes dans lequel l'agitation est de vitesse comprise dans la gamme de 1 à 2000 tr/min, de préférence réalisée au moyen d'un système d'agitation à pale, encore plus préférentiellement au moyen d'un système d'agitation à pale et contre-pale.

11. Poudre de polycondensat obtenue selon le procédé de l'une quelconque des revendications 1 à 10, **caractérisée en ce qu'**elle comprend des particules de poudre libre qui présentent une forme sphéroïdale, un D50 mesuré selon la norme ISO 13320-1:1999 compris dans la gamme de 1 à 200 µm, et comprennent des traces de fins de chaînes esters, de préférence 10 à 4000 meq/kg de fins de chaînes esters sur le poids de polycondensat.

12. Poudre selon la revendication 11, **caractérisée en ce qu'**elle comprend de 0 à 50%, de préférence de 0,01 à 30%, de préférence de 0,01 à 20%, d'un catalyseur de polycondensation, en moles par rapport au nombre de moles de polycondensat.

13. Poudre selon l'une quelconque des revendications 11 à 12, **caractérisée en ce qu'**elle comprend des traces d'un solvant de polycondensation tel que défini à l'une quelconque des revendications 1 ou 8, de préférence de 10 à 10000 ppm de solvant de polycondensation.

14. Poudre selon l'une quelconque des revendications 11 à 13, **caractérisée en ce qu'**elle comprend des traces, de préférence de 0,01 à 5%, en poids par rapport au poids total de la poudre, d'alcool de formule R₃OH et/ou R₇OH, où R₃, R₇ représentent chacun une chaîne alkyle de 1 à 5 atomes de carbone.

15. Poudre selon l'une quelconque des revendications 11 à 14, **caractérisée en ce que** le polycondensat est un oligomère de masse molaire moyenne en nombre mesurée par RMN comprise dans la gamme de 300 g/mol à 5000 g/mol.

16. Poudre selon l'une quelconque des revendications 11 à 15, **caractérisée en ce que** le polycondensat est un polyamide de masse molaire moyenne en nombre comprise entre 5000 à 30000 g/mol, de préférence comprise dans la gamme de 8000 à 15000 g/mol.

17. Utilisation de poudre selon l'une quelconque des revendications 11 à 16, dans les revêtements, les peintures, les compositions anticorrosion, les additifs pour papier, l'agglomération de poudre par fusion ou frittage provoqué par un rayonnement électromagnétique pour fabriquer des objets, les gels d'électrophorèse, les matériaux composites multicouches, l'emballage, les jouets, le textile, l'automobile et/ou l'électronique, dans les produits cosmétiques, pharmaceutiques ou de parfumerie.

18. Utilisation de poudre d'oligomère selon la revendication 15, comme synthon réactif ester et/ou amine dans les réactions d'allongement de chaîne de polymère, soit seul soit comme additif pour l'agglomération de poudre par fusion ou frittage provoqué par un rayonnement électromagnétique pour fabriquer des objets ou comme renfort de polyamide.

19. Dispersion de poudre d'oligomère ou de polymère obtenue selon le procédé de l'une quelconque des revendications 1 à 10, **caractérisée en ce qu'**elle comprend :
- de 0,1 % à 99,9% en poids, de préférence de 0,1 à 30% en poids, de particules de poudre conforme à l'une quelconque des revendications 11 à 16 ; et
- de 0,1% à 99,9% en poids, de préférence de 70 à 99,9% en poids, de solvant conforme à l'une quelconque des revendications 1 ou 8,
sur le poids total de la dispersion.

20. Composition cosmétique et/ou de parfumerie **caractérisée en ce qu'**elle comprend :
- de 0,1 à 99,9%, de préférence de 0,1 à 30% en poids, de particules de poudre telle que définie dans l'une quelconque des revendications 11 à 16, et
- de 0,1 à 99,9%, de préférence de 70 à 99,9% en poids, d'un milieu acceptable en cosmétique et/ou en parfumerie,
sur le poids total de la composition.

21. Composition selon la revendication 20, ladite composition étant un produit coloré, non coloré ou transparent choisi parmi les produits suivants :
- produits de maquillage pour le visage et le corps humain, tels que fond de teint, crème teintée, poudre libre ou compacte, fard à paupières, mascara, eye liner, rouge à lèvre ;
- produits de soins pour le visage et le corps humain, tels que crème, lait, lotion, masque, produit de gommage, produits nettoyants et/ou démaquillants, déodorants, antitranspirants, antiperspirants, produits de rasage, produits d'épilation ;
- produits capillaires, tels que shampooings, produits pour la mise en forme des cheveux, produits de maintien de la coiffure, produits antipelliculaires, produits antichute, produits contre la sécheresse des cheveux, teintures capillaires, produits de décoloration ;
- produits de parfumerie, tels que parfum, lait, crème, poudre libre ou compacte parfumée.

## Patentansprüche

1. Verfahren zur Herstellung einer Polykondensatpulver-Dispersion, **dadurch gekennzeichnet, dass** es mindestens einen Polykondensationsschritt:
i) mindestens eines Diesters und mindestens eines Diamins
und/oder
ii) mindestens eines Aminoesters
unter Rühren in einem Lösungsmittel, das sowohl das Diamin als auch den Diester und/oder den Aminoester, aber nicht das bei der Polykondensation gebildete Polyamid lösen kann, bei einer Temperatur im Bereich von 50 °C bis 120 °C und in Gegenwart von Siliciumdioxid so umfasst, dass ein Niederschlag eines im Lösungsmittel dispergierten Pulvers erhalten wird, wobei das Polykondensat in Form von Pulver isoliert wird.

2. Verfahren zur Herstellung einer Pulverdispersion nach Anspruch 1, **dadurch gekennzeichnet, dass** es außerdem einen Schritt der Abtrennung des Lösungsmittels und der Isolierung des Pulvers umfasst.

3. Verfahren nach einem der Ansprüche 1 oder 2, wobei die Polykondensation in Gegenwart eines Polykondensationskatalysators erfolgt, dessen Gehalt im Bereich von 0,01 bis 50 Mol-%, vorzugsweise von 0,01 bis 30 Mol-%, bezogen auf die Stoffmenge aller Reagenzien, liegt.

4. Verfahren nach einem der Ansprüche 1 bis 3, wobei der mindestens eine Diester die Formel:
R₁-(CH₂)ₘ-R₂, wobei m eine ganze Zahl von 0 bis 36 darstellt und R₁, R₂ identische oder verschiedene Esterfunktionen der allgemeinen Formel COOR₃ darstellen, wobei R₃ eine gesättigte oder ungesättigte, lineare oder verzweigte Alkylkette mit 1 bis 5 Kohlenstoffatomen darstellt,
und/oder
Rᵢ-(C₆H₄)ₙ-R₂ aufweist, wobei n eine ganze Zahl von 1 bis 2 darstellt und R₁, R₂ identische oder verschiedene Esterfunktionen der allgemeinen Formel COOR₃ darstellen, wobei R₃ eine gesättigte oder ungesättigte, lineare oder verzweigte Alkylkette mit 1 bis 5 Kohlenstoffatomen darstellt.

5. Verfahren nach einem der Ansprüche 1 bis 4, wobei das mindestens eine primäre oder sekundäre Diamin aus aliphatischen Diaminen mit 6 bis 12 Kohlenstoffatomen ausgewählt ist, wobei das Diamin ein Aryl- oder ein cyclisches gesättigtes Diamin sein kann.

6. Verfahren nach einem der Ansprüche 1 bis 5, wobei der mindestens eine Aminoester der allgemeinen Formel R₅-(CH₂)ₚ-R₆ entspricht, wobei p eine ganze Zahl von 0 bis 36 darstellt, R₅ eine primäre oder sekundäre Aminfunktion darstellt, R₆ eine Esterfunktion der allgemeinen Formel COOR₇ darstellt, wobei R₇ eine Alkylkette mit 1 bis 5 Kohlenstoffatomen darstellt.

7. Verfahren nach einem der Ansprüche 3 bis 6, wobei der Polykondensationskatalysator aus Natriumhydrid, Kaliumhydrid, Natrium, Natriumstearat, Orthophosphorsäure, Stearinsäure, Ethanol, Phenol, Natriummethylat, Natriumethylat und deren Mischungen ausgewählt ist.

8. Verfahren nach einem der vorhergehenden Ansprüche, wobei das Lösungsmittel aus linearen Alkanen, cycloaliphatischen Alkanen, halogenierten Lösungsmitteln und deren Mischungen ausgewählt ist, wobei das Lösungsmittel vorzugsweise eine Siedetemperatur im Bereich von 30 bis 180 °C aufweist.

9. Verfahren nach einem der vorhergehenden Ansprüche, wobei die Polykondensation durch ein gleichzeitiges Ausfallen in Form von Polykondensatpulvern gekennzeichnet ist.

10. Verfahren nach einem der vorhergehenden Ansprüche, wobei das Rühren bei einer Drehzahl im Bereich von 1 bis 2000 U/min erfolgt, wobei es vorzugsweise mittels eines Blattrührsystems, noch bevorzugter mittels eines Rührsystems mit gegenläufigen Blättern, erfolgt.

11. Polykondensatpulver, das gemäß dem Verfahren nach einem der Ansprüche 1 bis 10 erhalten wird, **dadurch gekennzeichnet, dass** es Partikel von freiem Pulver umfasst, die eine Kugelform, einen gemäß der Norm ISO 13320-1:1999 gemessenen D50 im Bereich von 1 bis 200 µm aufweisen und Spuren von Esterkettenenden, vorzugsweise 10 bis 4000 mVal/kg Esterkettenenden, bezogen auf das Gewicht des Polykondensats, umfassen.

12. Pulver nach Anspruch 11, **dadurch gekennzeichnet, dass** es von 0 bis 50 Mol-%, vorzugsweise von 0,01 bis 30 Mol-%, vorzugsweise von 0,01 bis 20 Mol-% eines Polykondensationskatalysators, bezogen auf die Stoffmenge des Polykondensats, umfasst.

13. Pulver nach einem der Ansprüche 11 bis 12, **dadurch gekennzeichnet, dass** es Spuren eines Polykondensationslösungsmittels gemäß der Definition in einem der Ansprüche 1 oder 8, vorzugsweise von 10 bis 10 000 ppm Polykondensationslösungsmittel, umfasst.

14. Pulver nach einem der Ansprüche 11 bis 13, **dadurch gekennzeichnet, dass** es Spuren, vorzugsweise von 0,01 bis 5 Gew.-%, bezogen auf das Gesamtgewicht des Pulvers, eines Alkohols der Formel R₃OH und/oder R₇OH umfasst, wobei R₃, R₇ jeweils eine Alkylkette mit 1 bis 5 Kohlenstoffatomen darstellen.

15. Pulver nach einem der Ansprüche 11 bis 14, **dadurch gekennzeichnet, dass** das Polykondensat ein Oligomer mit einem mittels NMR gemessenen Zahlenmittel der Molmasse im Bereich von 300 g/mol bis 5000 g/mol ist.

16. Pulver nach einem der Ansprüche 11 bis 15, **dadurch gekennzeichnet, dass** das Polykondensat ein Polyamid mit einem Zahlenmittel der Molmasse zwischen 5000 und 30 000 g/mol, vorzugsweise im Bereich von 300 g/mol bis 15 000 g/mol, ist.

17. Verwendung des Pulvers nach einem der Ansprüche 11 bis 16 in Beschichtungen, Anstrichmitteln, Korrosionsschutzzusammensetzungen, Additiven für Papier, einer durch elektromagnetische Strahlung bewirkten Pulveragglomeration durch Schmelzen oder Sintern zur Herstellung von Gegenständen, Elektrophoresegelen, mehrlagigen Verbundwerkstoffen, Verpackungen, Spielzeug, einem Textilerzeugnis, einem Kraftfahrzeug und/oder in der Elektronik, in kosmetischen, pharmazeutischen oder Parfümerieprodukten.

18. Verwendung des Oligomerpulvers nach Anspruch 15 als reaktives Ester- und/oder Amin-Synthon in Reaktionen zur Polymerkettenverlängerung entweder allein oder als Additiv für eine durch elektromagnetische Strahlung bewirkte Pulveragglomeration durch Schmelzen oder Sintern zur Herstellung von Gegenständen oder als Polyamidverstärkung.

19. Oligomer- oder Polymer-Pulverdispersion, die gemäß dem Verfahren nach einem der Ansprüche 1 bis 10 erhalten wird, **dadurch gekennzeichnet, dass** sie Folgendes umfasst:
- 0,1 Gew.-% bis 99,9 Gew.-%, vorzugsweise 0,1 bis 30 Gew.-% Pulverpartikel nach einem der Ansprüche 11 bis 16 und
- 0,1 Gew.-% bis 99,9 Gew.-%, vorzugsweise 70 bis 99,9 Gew.-% Lösungsmittel nach einem der Ansprüche 1 oder 8,
bezogen auf das Gesamtgewicht der Dispersion.

20. Kosmetische und/oder Parfümerie-Zusammensetzung, **dadurch gekennzeichnet, dass** sie Folgendes umfasst:
- 0,1 Gew.-% bis 99,9 Gew.-%, vorzugsweise 0,1 bis 30 Gew.-% Pulverpartikel gemäß der Definition in einem der Ansprüche 11 bis 16 und
- 0,1 bis 99,9 Gew.-%, vorzugsweise 70 bis 99,9 Gew.-% eines in der Kosmetik und/oder der Parfümerie annehmbaren Mediums,
bezogen auf das Gesamtgewicht der Zusammensetzung.

21. Zusammensetzung nach Anspruch 20, wobei es sich bei der Zusammensetzung um ein gefärbtes, nicht gefärbtes oder transparentes Produkt handelt, das aus den folgenden Produkten ausgewählt ist:
- Schminkprodukten für das menschliche Gesicht und den menschlichen Körper, wie eine Grundierung, getönte Creme, loser oder Kompaktpuder, Lidschatten, Maskara, Eyeliner, Lippenstift;
- Pflegeprodukte für das menschliche Gesicht und den menschlichen Körper, wie eine Creme, Milch, Lotion, Maske, ein Peeling-Produkt, Reinigungs- und/oder Abschminkprodukte, Deodorants, Antitranspirantien, Antiperspirantien, Rasierprodukte, Haarentfernungsprodukte;
- Haarpflegeprodukte wie Shampoos, Produkte zur Haarformung, Produkte zur Erhaltung der Frisur, Antischuppenprodukte, Produkte gegen Haarausfall, Produkte gegen Haartrockenheit, Haarfärbemittel, Entfärbungsprodukte;
- Parfümerieprodukte, wie ein Parfüm, eine Milch, Creme, einen parfümierten losen oder Kompaktpuder.

## Claims

1. Process for producing a polycondensate powder dispersion, **characterized in that** it comprises at least one step of polycondensation:
i) of at least one diester and at least one diamine, and/or
ii) of at least one amino ester,
with stirring, in a solvent that can dissolve both the diamine and the diester and/or the amino ester, but not the polyamide which forms during the polycondensation, at a temperature included in the range of from 50°C to 120°C and in the presence of silica, such that a powder precipitate dispersed in the solvent is obtained, and the polycondensate is recovered in powder form.

2. Process for producing a powder dispersion according to Claim 1, **characterized in that** it also comprises a step of separation of the solvent and of recovery of the powder.

3. Process according to either one of Claims 1 and 2, wherein said polycondensation is carried out in the presence of a polycondensation catalyst, the content of which is included in the range of from 0.01 mol% to 50 mol%, preferably from 0.01 mol% to 30 mol%, relative to the number of moles of all of the reagents.

4. Process according to any one of Claims 1 to 3, wherein said at least one diester is of formula:
R₁-(CH₂)ₘ-R₂, wherein m represents an integer ranging from 0 to 36, and R₁ and R₂ represent identical or different ester functions of general formula COOR₃, wherein R₃ represents a saturated or unsaturated, linear or branched alkyl chain of from 1 to 5 carbon atoms,
and/or
R₁-(C₆H₄)ₙ-R₂, wherein n represents an integer ranging from 1 to 2, and R₁ and R₂ represent identical or different ester functions of general formula COOR₃, wherein R₃ represents a saturated or unsaturated, linear or branched alkyl chain of from 1 to 5 carbon atoms.

5. Process according to any one of Claims 1 to 4, wherein said at least one primary or secondary diamine is chosen from aliphatic diamines having from 6 to 12 carbon atoms, said diamine possibly being a saturated aryl or cyclic diamine.

6. Process according to any one of Claims 1 to 5, wherein said at least one amino ester corresponds to the general formula R₅-(CH₂)ₚ-R₆, wherein p represents an integer ranging from 0 to 36, R₅ represents a primary or secondary amine function, and R₆ represents an ester function of general formula COOR₇, wherein R₇ represents an alkyl chain of from 1 to 5 carbon atoms.

7. Process according to any one of Claims 3 to 6, wherein the polycondensation catalyst is chosen from sodium hydride, potassium hydride, sodium, sodium stearate, ortho-phosphoric acid, stearic acid, ethanol, phenol, sodium methoxide, sodium ethoxide, and mixtures thereof.

8. Process according to any one of the preceding claims, wherein the solvent is chosen from linear alkanes, cycloaliphatic alkanes, halogenated solvents and mixtures thereof, the solvent preferably having a boiling point included in the range of from 30°C to 180°C.

9. Process according to any one of the preceding claims, wherein the polycondensation is **characterized by** a simultaneous precipitation in the form of polycondensate powders.

10. Process according to one of the preceding claims, wherein the stirring is at a speed included in the range of from 1 to 2000 rpm, preferably carried out by means of a paddle stirring system, even more preferably by means of a paddle and counter-paddle stirring system.

11. Polycondensate powder obtained according to the process of any one of Claims 1 to 10, **characterized in that** it comprises particles of free powder which have a spheroidal shape, a D50 measured according to ISO standard 13320-1:1999 included in the range of from 1 to 200 µm, and which comprise traces of ester chain ends, preferably 10 to 4000 meq/kg of ester chain ends, relative to the weight of polycondensate.

12. Powder according to Claim 11, **characterized in that** it comprises from 0 to 50 mol%, preferably from 0.01 mol% to 30 mol%, preferably from 0.01 mol% to 20 mol%, of a polycondensation catalyst, relative to the number of moles of polycondensate.

13. Powder according to either one of Claims 11 and 12, **characterized in that** it comprises traces of a polycondensation solvent as defined in either one of Claims 1 and 8, preferably from 10 to 10 000 ppm of polycondensation solvent.

14. Powder according to any one of Claims 11 to 13, **characterized in that** it comprises traces, preferably from 0.01% to 5% by weight, relative to the total weight of the powder, of alcohol of formula R₃OH and/or R₇OH, wherein R₃ and R₇ each represent an alkyl chain of from 1 to 5 carbon atoms.

15. Powder according to any one of Claims 11 to 14, **characterized in that** the polycondensate is an oligomer having a number-average molar mass measured by NMR included in the range of from 300 g/mol to 5000 g/mol.

16. Powder according to any one of Claims 11 to 15, **characterized in that** the polycondensate is a polyamide having a number-average molar mass of between 5000 and 30 000 g/mol, preferably included in the range of from 8000 to 15 000 g/mol.

17. Use of powder according to any one of Claims 11 to 16, in coatings, paints, anticorrosion compositions, paper additives, powder agglomeration by electromagnetic radiation-induced melting or sintering for producing objects, electrophoresis gels, multilayer composite materials, packaging, toys, the textile, automobile and/or electronics industry, and in cosmetic, pharmaceutical or perfumery products.

18. Use of oligomer powder according to Claim 15, as an ester and/or amine reactive synthon in polymer chain extension reactions, either alone or as an additive for powder agglomeration by electromagnetic radiation-induced melting or sintering for producing objects or as a polyamide reinforcement.

19. Oligomer or polymer powder dispersion obtained according to the process of any one of Claims 1 to 10, **characterized in that** it comprises:
- from 0.1% to 99.9% by weight, preferably from 0.1% to 30% by weight, of particles of powder in accordance with any one of Claims 11 to 16; and
- from 0.1% to 99.9% by weight, preferably from 70% to 99.9% by weight, of solvent in accordance with either one of Claims 1 and 8,
relative to the total weight of the dispersion.

20. Cosmetic and/or perfumery composition, **characterized in that** it comprises:
- from 0.1% to 99.9%, preferably from 0.1% to 30% by weight, of particles of powder as defined in any one of Claims 11 to 16, and
- from 0.1% to 99.9%, preferably from 70% to 99.9% by weight, of a medium which is acceptable in cosmetics and/or in perfumery,
relative to the total weight of the composition.

21. Composition according to Claim 20, said composition being a coloured, noncoloured or transparent product chosen from the following products:
- makeup products for the human face and body, such as a foundation, tinted cream, loose or compact powder, eyeshadow, mascara, eyeliner or lipstick;
- care products for the human face and body, such as a cream, milk, lotion, mask, exfoliation product, cleansing and/or makeup-removal products, deodorants, antiperspirants, shaving products or hair-removal products;
- hair products, such as shampoos, hair shaping products, hairstyle maintaining products, antidandruff products, anti-hair loss products, products for counteracting dryness of the hair, hair dyes or bleaching products;
- perfumery products, such as a fragrance, milk, cream or loose or compact powder which is fragranced.
